(19) Europäisches Patentamt
European Patent Office
Office européen des brevets

(11) EP 0 484 785 B1

(12) **EUROPEAN PATENT SPECIFICATION**

(45) Date of publication and mention of the grant of the patent:
**22.05.1996 Bulletin 1996/21**

(51) Int. Cl.$^6$: **A61K 31/52**

(21) Application number: **91118347.3**

(22) Date of filing: **28.10.1991**

(54) **Use of xanthines for the preparation of a medicament effective for inhibiting the replication of human retroviruses**

Verwendung von Xanthinen zur Herstellung von Arzneimitteln zur Hemmung der Vermehrung menschlicher Retroviren

Utilisation de xanthines pour la préparation de médicaments actifs comme inhibiteurs de la réplication des retrovirus humains

(84) Designated Contracting States:
**AT BE CH DE DK ES FR GB GR IT LI LU NL SE**

(30) Priority: **07.11.1990 US 610230**

(43) Date of publication of application:
**13.05.1992 Bulletin 1992/20**

(73) Proprietor: **HOECHST-ROUSSEL PHARMACEUTICALS INCORPORATED**
**Somerville New Jersey 08876 (US)**

(72) Inventors:
- **Dezube, Bruce J.**
  **Newton Centre, MA 02159 (US)**
- **Pardee, Arthur B.**
  **Brookline, MA 02146 (US)**
- **Ruprecht, Ruth M.**
  **Boston, MA 02215 (US)**
- **Novick, William J., Jr.**
  **Lebanon, NJ 08833 (US)**

(74) Representative: **Aulmich, Gerhard, Dr. et al**
**Hoechst AG**
**Patent- und Lizenzabteilung**
**Gebäude K 801**
**D-65926 Frankfurt am Main (DE)**

(56) References cited:
**EP-A- 0 232 438** **EP-A- 0 243 192**
**EP-A- 0 305 743** **US-A- 4 663 317**

- **BIOCHEMICAL AND BIOPHYSICAL RESEARCH COMMUNICATIONS, vol. 155, no. 3, September 30, 1988, pages 1230-1236, Academic Press, Inc.; R.D. STRIETER et al.: "Cellular and molecular regulation of tumor necrosis factor-alpha production by pentoxifylline",**
- **PROC. NATL. ACAD. SCI, USA, vol. 86, August 1989, Microbiology, pages 5974-5978, E.J. DUH et al.: "Tumor necrosis factor alpha activates human immunodeficiency virus type 1 through induction of nuclear factor binding to the NF-kB sites in the long terminal repeat",**
- **J.Aids, G, 787-94 (1993)**
- **Int. J.Clin. Pharmacol. Ther. Taxicol., 31, 343-50 (1993)**

Remarks:
The file contains technical information submitted after the application was filed and not included in this specification

EP 0 484 785 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.11.7/3.4

## Description

BACKGROUND OF THE INVENTION

This invention relates to the use of xanthine compounds of the formula (II) in the preparation of a medicament for inhibiting the replication of human retroviruses, such as human immunodeficiency virus type 1 (HIV-1). This invention also relates to a use of xanthine compounds of the formula (II) in preparation of a medicament for inhibiting proliferation of human retroviruses as measured by reduction in reverse transcriptase activity.

Acquired immune deficiency syndrome (AIDS) is a condition that is now of major importance in North America, Europe and Central Africa. The causative agent of AIDS is believed to be a retrovirus, namely HIV-1. Recent estimates suggest that at least 1.5 million Americans may have been exposed to the AIDS virus called HIV-1, and that by 1991, 15 million people in the United States will have been infected with the virus. The individuals affected show severe immunosuppression, which may be followed by the onset of degenerative and even fatal diseases.

The isolation and characterization of the first AIDS retrovirus, known as LAV and later termed HIV-$1_{LAV}$, was described in a paper by F. Barre-Sinoussi, et al. Science, 220 : 868 - 871 (1983). The use of some extracts of this virus and some of its proteins to detect antibodies against the virus is described in U. S. Patent No. 4,708,818.

Several isolates of the AIDS retrovirus were subsequently reported by different investigators and the isolates were referred to in the literature by different designations. It is now universally recognized that viruses previously denominated lymphadenopathy associated virus (LAV), immune deficiency associated virus (IDAV1 and IDAV2), human T-lymphotropic virus type III (HTLV-III), and AIDS related virus (ARV) are all variants of the same retrovirus. See, e. g., Nature, 313 : 636 - 637 (1985).

A subcommittee empowered by the International Committee on the Taxonomy of Viruses proposed that the AIDS retroviruses be officially designated as the "Human Immunodeficiency Viruses", to be known in abbreviated form as "HIV". Isolates of human retroviruses with clear but limited relationship to isolates of HIV (for example, more than 20% but less than 50% nucleic acid sequence identity) are not to be called HIV unless there are compelling biological and structural similarities to existing members of the group. Science, 232:697 (1986).

Another pathogenic human retrovirus, termed HIV-2 (formerly LAV-2), was recovered from West African patients with AIDS. Clavel et al., Science, 233:343-346 (1986). HIV-2 infection is associated with an immunodeficiency syndrome clinically indistinguishable from that caused by the prototype AIDS virus, HIV-1. HIV-2 is related to but distinct from HIV-1 and induced immunodeficiency syndrome with a markedly lower frequency. Guyader et al., Nature, 326:662-669 (1987).

Retroviruses genetically related and biologically similar to HIV have been isolated from subhuman primates. These retroviruses are designated as immunodeficiency viruses of the appropriate host species, such as, simian immunodeficiency virus (SIV). SIV was first isolated from captive rhesus macaques (*Macaca mulatta*) at the New England Regional Primate Research Center (NERPRC). Kanki et al., Science **228**:1199 (1985). This was soon followed by a report of isolation of an SIV called STLV-III from African green monkeys. Kanki et al., Science **230**:951 (1985). Extensive serologic cross-reactivity exists between HIV-2 and SIV.

Transmission of HIV frequently takes place through sexual contact, although people using narcotics intravenously also represent a high-risk group. A large number of individuals have also been infected with HIV after receiving contaminated blood or blood products. In addition, HIV-infected pediatric patients have been born to mothers infected with HIV. The virus is believed to be transmitted from infected mothers to their fetuses.

The existence of multiple human immunodeficiency viruses, such as HIV-1 and HIV-2, presents a complex epidemiologic picture. HIV infection is now the number one public health menace in the United States and a leading cause of death in certain high population areas. It is known that pentoxifylline suppresses the production of lipopolysaccharides-induced monocytes-derived tumor necrosis factor (Biochem. Biophys. Res. Communication, 155 (3), (1988) pages 1230 - 1236) and that tumor necrosis factor and the supernatant of lipopolysaccharides-stimulated monocytes are potent activators of HIV production (Proc. Natl. Acad. Sci. USA, 86 (1989) pages 5974 - 5978).

EP 0 305 743 discloses the use of pentoxifylline in a combined preparation with sulfated polysaccharides for the treatment of retrovirus disease.

EP 0 232 438 discloses the use of xanthine derivatives in the preparation of a medicament having the activity of enhancing host defense mechanism against trauma.

There is a common belief that an effective vaccine or pharmaceutical composition against HIV infection must be developed in order to stem the spread of these retroviruses. Work is progressing on the development of a vaccine, but an effective agent has not yet been found. Thus, there exists a need in the art for a method of inhibiting the activity of human retroviruses.

SUMMARY OF THE INVENTION

This invention aids in fulfilling these needs in the art. More particularly, this invention provides a use of xanthine compounds of the formula (II) in the preparation of a medicament for inhibiting the replication of human retroviruses,

such as human immunodeficiency virus (HIV). The use comprises administering to a human host a xanthine that is capable of exhibiting a protective effect, or of preventing retroviral replication.

This invention provides the use of a combined amount of

A) a compound of the formula II

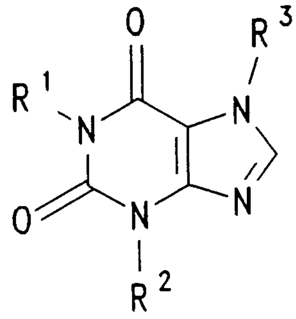

(II)

wherein

a) one of $R^1$ and $R^3$ is a branched hydroxyalkyl group of the formula

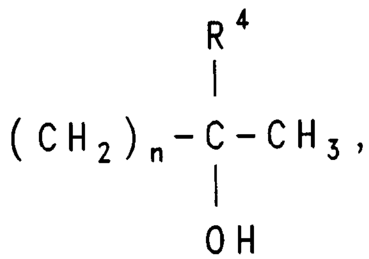

in which $R^4$ stands for an alkyl group with 1 to 3 carbon atoms and n stands for a whole number from 2 to 5, the other $R^1$ or $R^3$ group is a hydrogen atom or an aliphatic hydrocarbon group with up to 6 carbon atoms, whose carbon chain may be interrupted by up to 2 oxygen atoms or may be substituted with a hydroxy or oxo group, or

b) one of $R^1$ and $R^3$ is an oxoalkyl group of the formula

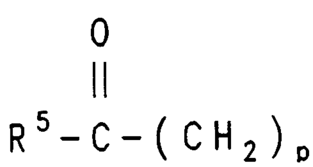

wherein $R^5$ is $C_1$-$C_6$ alkyl and p is 2, 3 or 4, the other $R^1$ or $R^3$ is a hydrogen atom or an aliphatic hydrocarbon group with up to 6 carbon atoms, whose carbon chain may be interrupted by up to 2 oxygen atoms or may be substituted with a hydroxy or oxo group, or

c) $R^1$ and $R^3$ are

I) a branched hydroxyalkyl group of the formula

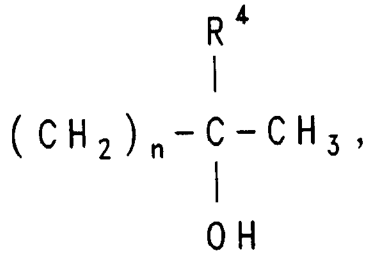

in which $R^4$ is as defined above or

II) an oxoalkyl group of the formula

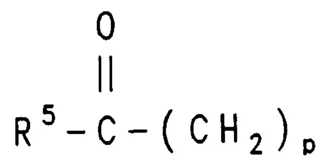

wherein $R^5$ is as defined above;

and $R^2$ is an $C_1$-$C_4$ alkyl group; and

B) an antiviral agent selected from the group consisting of 3'-azido-2',3'-dideoxythymidine (AZT); 2',3'-dideoxycytidine (ddC); 2',3'-dideoxyadenosine (ddA); and 2',3'-dideoxyinosine (ddI); or combinations of these agents;

for the preparation of a medicament effective in inhibiting replication of a human immunodeficiency virus.
The xanthine of formula (II) is employed in an amount that is effective in inhibiting replication of the retrovirus. The well known pharmaceutical compound pentoxifylline is an example of a compound within the general formula (II). Pentoxifylline is commercially available under the trademark Trental® in the form of tablets for oral administration. Although this compound has been used as a pharmaceutical to improve the flow properties of blood (clinical trials in 1971), it has not been reported effective as an inhibitor of retroviral replication.

The evasiveness and diversity of HIV has made a definitive treatment difficult. Presented here is an agent and a use capable of aiding in the prevention of the spread of HIV infection and of assisting in the treatment of such infection by administering the xanthine in a combination with an antiviral agent to a human in an amount sufficient to prevent or at least inhibit replication of HIV *in vivo*.

DETAILED DESCRIPTION OF PREFERRED EMBODIMENT

Certain xanthines are employed according to this invention to inhibit the replication of a human retrovirus. The nature of the biological processes involved in this invention initially will be described. This will be followed by a detailed description of the xanthines and methods for preparing the xanthines. The results obtained by *in vitro* testing will then be presented.

DETAILED DESCRIPTION OF PREFERRED EMBODIMENTS

The use of this invention can be employed to inhibit replication of a human retrovirus, which is an enveloped single-stranded RNA virus. The virus contains reverse transcriptase (RT), which is used to catalyze the transcription of the RNA genome of the virus into the DNA form known as the provirus. The DNA form typically integrates into host cell DNA. Preferably, the xanthines are employed as described in this invention to inhibit the activity of human immunodeficiency virus (HIV), including HIV-1 and HIV-2.

This invention is useful for the treatment of humans either infected with HIV or susceptible to HIV infection. The invention is especially useful for inhibiting the activity of HIV-1 or HIV-2 in humans infected therewith. While this invention is described with reference to human isolates identified in the literature as LAV-I, HTLV-III, and LAV-II, it will be understood that this invention extends to the same or equivalent retroviruses. These retroviruses are believed to be the causative agents of AIDS.

For the purpose of this disclosure, a virus is considered to be the same as or equivalent to LAV/HTLV-III if it substantially fulfills the following criteria:

(a) The virus is tropic for T-lymphocytes, especially T-helper cells ($CD4^+$);
(b) The virus is cytopathic for infected $CD4^+$ cells;
(c) The virus encodes an RNA-dependent DNA polymerase (reverse transcriptase), which is $Mg^{++}$ dependent and can employ poly$(A)_n$ oligo$(dT)_{12-18}$ as a template/primer for reverse transcription;
(d) The virus is substantially cross-reactive immunologically with the proteins encoded by the gag and env regions of LAV/HTLV-III; and
(e) The virus shares substantial nucleotide homology (approximately 75-100%) and amino acid sequence homology (approximately 75-100%) with LAV or HTLV-III.

The principal pharmaceutically active ingredient employed in the method of the invention is a xanthine. As used herein, the term "xanthine" refers to compounds of formula (II) that have a protective (prophylactic) effect against HIV infection, a curative (therapeutic) effect against HIV infection, or prevent transmission of HIV in humans. The term "xanthines" refers to compounds of the formula (II) that are used before HIV infection occurs or before HIV infection becomes evident with the aim of preventing or reducing the occurrence of the infection. The term "Xanthines" also refers to compounds of the formula (II) that are therapeutically used for their action on established HIV infection. Further, the term "xanthines" refers to compounds of the formula (II) that are useful for the prevention of infection of humans by HIV vectors, including xanthines of the formula (II) that intervene or interfere with the HIV replication cycle in a human host.

The mechanism by which the xanthines of this invention inhibit the replication of HIV *in vivo* is not entirely understood. The xanthines of the formula (II) have been shown to inhibit HIV-1 replication as measured by RT activity of the supernatants of infected cultures. Nevertheless, the mechanism of inhibiting the activity of human retroviruses may be due to one factor or to a combination of factors, such as a modification of one or more genotypic or phenotypic traits of the retrovirus or modification of viral or cellular processes. Moreover, there is evidence that the retrovirus enters the cell; the xanthine may alter the entry process. Further, the genomic elements that control the expression of the products required for viral replication may be altered or their functioning may be affected by the xanthine. Thus, for example, the xanthine may alter the nature of the envelope glycoprotein of the retrovirus or inhibits the expression of various genes. The xanthines may also operate by mediating the functioning of the reticuloendothelial system, either before or after HIV infection has occurred. The xanthines may also inhibit proliferation of HIV in infected phagocytic cells. It will be understood that the xanthines of the formula (II) may function in any of these ways or by combinations thereof or by other ways not currently recognized. In any event, the xanthines of the formula (II) can be administered to the patient in sufficient amounts to achieve one or more of these effects.

The use of the xanthines according to the invention in patients with AIDS is predicated on inhibition of HIV replication, which will result in some regeneration of the host and at least reduce further deterioration of the immune system of the host. The effectiveness of the xanthines in preventing or inhibiting viral replication and infection of cells can be demonstrated using standard *in vitro* assays. For example, the inhibitory effect of the xanthines on HIV infection or replication can be demonstrated by cultivating the virus or virus-infected cells in the presence and in the absence of the xanthine and then comparing the results.

The ability of the xanthines to inhibit the replication of a human retrovirus can be demonstrated *in vitro* by measuring reverse transcriptase activity. For example, conditioned media from retrovirus infected cells can be concentrated by polyethylene glycol precipitation or by centrifugation. Synthetic template primers containing 12 to 18 bases of a DNA primer can be annealed to 300 bases of RNA template. Virons containing RT can be disrupted by a detergent, such as Triton X-100. In the presence of an appropriately labeled nucleoside triphosphate, such as radioactively labeled guanosine triphosphate, a divalent cation (usually $Mg^{++}$), and potassium, the RT can efficiently incorporate the labeled nucleotide into the DNA strand as directed by the nucleotide sequence of the RNA strand. This results in a acid precipitatable, radioactively labeled RNA:DNA hybrid. This product can be analyzed in a liquid scintillation counter and the results expressed as picomoles of nucleoside monophosphate incorporated per unit time, or if the results are compared to a standard, as RT units. Two particular synthetic template-primers are routinely used in this assay: Poly$(A)_n$ oligo$(dT)_{12-18}$ and poly$(C)_n$ oligo(dG). The template/primer poly$(C)_n$ oligo$(dG)_{12-18}$ is retrovirus specific and is utilized efficiently by human retroviral reverse transcriptase in the presence of $Mg^{++}$.

Inhibition of HIV-1 replication also be detected using monoclonal or polyclonal antibodies in an immunoassay. More particularly, certain immunoassays can be employed to screen the xanthines of the invention for efficacy in *in vitro* and

*in vivo* studies. The assays include competitive enzyme linked immunosorbent assay (ELISA) and antigen capture assays.

The polymerase chain reaction (PCR) can also be employed as an assay for testing the inhibition of HIV-1 replication by the *in vitro* enzymatic amplification of small regions of the human retroviruses, e.g. the RT or *gag* regions. Since the nucleotide sequence encoding RT is known for human retroviruses, the sequence information can be used to synthesize oligonucleotides of 20 to 30 bases for use as primers in DNA synthesis in PCR. The DNA from the cells under investigation can be added to the primer oligonucleotides in the presence of all four nucleoside triphosphates, a heat-stable DNA-dependent DNA polymerase, and an appropriate buffer. The reaction mixture can then be cycled through the conventional three-stage amplification procedure, involving heat denaturation of the DNA, annealing of the primers to the resulting single-stranded DNA, and primer extension to form multiple copies of the target sequence. With each cycle there is a doubling of the target DNA, resulting in a geometric expansion of the target sequence.

Several methods exist to quantitate the amplified products. For primer pairs in which there is minimal DNA synthesis in normal samples, it is possible to use radioactively labeled nucleoside triphosphates as substrates and acid precipitate the amplified product and analyze the product in a liquid scintillation counter. If there is significant DNA synthesis occurring in normal DNA for a given primer pair, the product can be fractionated on a mini scale and specific bands quantitated using a densitometer. Similarly, spot blots or Southern blots on the amplified product can be performed, and the amount of hybridized detector can be quantified.

Another method for demonstrating the effectiveness of the xanthines in inhibiting retroviral replication according to the invention involves cultivation of normal peripheral blood mononuclear cells or other cultured cells exposed to the virus. The test for infectivity can use either free virus or virus-infected cells that are co-cultivated with a sensitive indicator cell. The virus replicates in the recipient indicator cell. By comparing viral replication in the absence of the xanthine with viral replication in the presence of the drug, the inhibiting effect of the xanthine on HIV can be demonstrated.

The inhibition of retroviral replication according to the invention can also be demonstrated by examining virus production as determined by electron microscopic evidence of virus particles and various viral proteins other than RT. Various detection protocols for viral proteins make use of standard antibodies prepared against the virus in many animal species. Immunofluorescence can detect viral proteins rapidly and sensitive hyperimmune sera can be prepared. Radioimmune and ELISA assays can also be used to measure presence of viral proteins by competition in a system where a radiolabeled or colorimetrically identifiable antigen-antibody system can be perturbed by the addition of reactive antigen.

Another approach for demonstrating the effectiveness of the xanthines according to the invention involves detection of the virus by detecting unintegrated and integrated viral DNA as well as viral mRNA. Nature, 312:166-169 (1984). More particularly, in a typical experiment, cells are exposed to cell-free virions at a multiplicity of viral particles per cell and cultured in the presence or absence of xanthines. High molecular weight DNA is extracted at various times and assayed for its content of viral DNA using a radiolabelled HIV probe. In the absence of xanthines under the culture conditions, viral DNA is detected. In contrast, in DNA from cells that have been completely protected by xanthines, neither unintegrated nor integrated DNA is detected.

Southern and Northern blot hybridization techniques are useful in determination of the relative amounts of viral DNA and RNA of the virus-harboring cells and tissues. Science, 227:177-182 (1985). A probe can be constructed for integrated provirus using molecularly cloned labeled proviral DNA, and then one can determine in a DNA transfer experiment whether there is a proviral genome integrated in cellular DNA by hybridization. If only a few cells contain the provirus, *in situ* hybridization can be attempted. These techniques can be employed to demonstrate the effect of the xanthines on replication of the retrovirus.

It is also possible to monitor viral replication by determining whether viral RNA is expressed in target T cells exposed to the virus and cultured with or without xanthines. In this experiment, cells are exposed to HIV, and RNA is extracted from the cells. Extracted RNA is then assayed for the content of viral mRNA by Northern blot hybridization using a radiolabelled HIV antisense RNA probe. Science, 227:177-182 (1985). In the absence of inhibitor, viral mRNA is detectable. When these cultures are maintained for extensive periods of time in the presence of xanthines, viral RNA expression in the cells is not detected, or if detected, is present in reduced amount. This assay system makes it possible to assess the capacity of the xanthines to inhibit HIV nucleotide synthesis and mRNA expression in T cells exposed to the virus.

In addition, HIV-1-infected cell lines MT2 and MT4 are sensitive to the cytopathic effect of the virus. Thus, these cells can be used in plaque-forming assays to demonstrate the inhibitory effect of the xanthines on HIV infection or proliferation. Science, 229:563-566 (1985).

The effectiveness of the xanthines in inhibiting HIV infection or HIV replication can also be demonstrated *in vitro* by determining the inhibitory effect of the drugs on viral p24 gag protein expression in H9 cells, partially resistant to the cytopathic effect of HIV. M. Popovic et al., Science, 224:497-500 (1984). Anti-HIV activity can also be demonstrated by showing the inhibitory effect of the xanthines on the cytopathic effect of HIV. M. Hiroaki et al., Science, 240:646-649 (1988).

Finally, the activity of the xanthines can be shown in normal T cells *in vitro*. In this assay, normal cloned helper-inducer T cells, such as normal tetanus toxid-specific helper/inducer clonal T cells (TMII cells), are used to monitor the

effect of the xanthines on antigen-induced proliferative response. Details of this technique are described by H. Mitsuya et al., Science, 240:646-649 (1988).

The effectiveness of the xanthines in preventing or inhibiting HIV infection or replication can be confirmed *in vivo* with the chimpanzee or in chimeric SCID mice reconstituted with cells of the human immune system. Persistent infection with HIV is demonstrated with this primate, although AIDS-like disease is not shown. The effectiveness of the xanthines can be demonstrated by comparing treated and untreated chimpanzees for seroconversion to HIV antigens, by reisolating infectious virus from the animals, by demonstrable lymphadenopathy, by alterations in T4 or T8 lymphocyte levels, or by combinations of these measures.

HIV required to carry out these assays can be obtained from conventional sources using known techniques. For instance, mononuclear cells prepared from peripheral blood, bone marrow, and other tissues from patients and donors can be stimulated with mitogen (phytohemagglutinin-PHA) for 48 to 72 hrs and established in cell culture using growth medium supplemented with T cell growth factor (TCGF). Virus can be detected by: (1) monitoring supernatant fluids for viral reverse transcriptase activity; (2) transmitting virus to fresh normal human T-lymphocytes (e.g., umbilical cord blood, adult peripheral blood, or bone marrow leukocytes) or to established T-cell lines; M. Popovic et al., Science, 224:497 (1984); (3) electron microscopic observation of fixed and sectioned cells; and (4) testing for antigen expression by indirect immunofluorescence or Western blot procedures using serum from seropositive donors. The detection of virus-positive cells and the characterization and comparison of viral isolates can be conducted using HIV-specific immunologic reagents and nucleic acid probes. F. Barre-Sinoussi et al., Science, 220:868-871 (1983); R. C. Gallo et al., Science, 220:865-867 (1983).

Many established cell lines have been tested as possible targets for HIV-1 infection. These cell lines can also be employed to demonstrate the effectiveness of the xanthines in the assays described above. One neoplastic aneuploid T-cell line derived from an adult with lymphoid leukemia and termed HT is susceptible to infection with HTLV-III. HT cells continuously produce HTLV-III after parental cells are repeatedly exposed to concentrated cell culture fluids harvested from short-term cultured T-cells (grown in TCGF) that originated from patients with lymphadenopathy syndrome or AIDS. When cell proliferation declines, usually 10 to 20 days after exposure to the culture fluids, the fresh (uninfected) HT parental cells are added to cultures. To improve permissiveness for HIV and to preserve permanent growth and continuous production of virus, the HT cell population has been extensively cloned. Several of these clones of HT cells have been maintained in cell culture.

In addition to HT, there are several other T or pre-T human cell lines that can be infected by and continue to produce HIV. Examples of these cell lines are H4, H9, HUT 78, CEM, Molt 3, and Ti7.4. Gallo et al., U.S. Patent 4,652,599. Furthermore, some B-lymphoblastic cell lines can also be productively infected by HIV. Montagnier et al., Science, 225:63-66 (1984).

In summary, the foregoing procedures are examples of the techniques that can be employed to demonstrate the effectiveness of the xanthines in inhibiting replication of a human retrovirus. Specific examples of the inhibition of retroviral replication by the xanthines are included, *infra*.

2. Administration of Xanthines to Human Patients

A xanthine or a mixture of the xanthines can be administered, in combination with certain antiviral agents, to a human subject in need of therapy in an amount sufficient to decrease replication of a human retrovirus. While mixtures of xanthines can be employed, no particular advantage has been observed in the use of mixtures.

The xanthines and their pharmaceutically acceptable salts can be used in mammalian, including but not limited to human, therapy in the form of pills, tablets, lozenges, troches, capsules, suppositories, injectable or ingestable solutions and the like, in the treatment of humans and susceptible non-human primates caused by disturbances in the functioning of the immune system.

The xanthines, while effective themselves, can be formulated and administered in the form of their pharmaceutically acceptable addition salts for purposes of stability, convenience of crystallization, increased solubility, and the like. Preferred pharmaceutically acceptable addition salts include salts of mineral acids, for example, hydrochloric acid, sulfuric acid, nitric acid, and the like; salts of monobasic carboxylic acids, such as, for example, acetic acid, propionic acid, and the like; salts of dibasic carboxylic acids, such as, maleic acid, fumaric acid, oxalic acid, and the like; and salts of tribasic carboxylic acids, such as, carboxysuccinic acid, citric acid, and the like.

The xanthines can be administered orally, for example, with an inert diluent or with an edible carrier. They can be enclosed in gelatin capsules or compressed into tablets. For the purpose of oral therapeutic administration, the compounds can be incorporated with excipients and used in the form of tablets, troches, capsules, elixirs, suspensions, syrups, wafers, chewing gums, and the like. These preparations should contain at least 0.5% of active compound, but the amount can be varied depending upon the particular form and can conveniently be between 4.0% to about 70% of the weight of the unit. The amount of xanthine in such compositions is such that a suitable dosage will be obtained. Preferred compositions and preparations according to the present invention are prepared so that an oral dosage unit form contains between about 1.0 mgs and about 400 mgs of active compound.

Tablets, pills, capsules, troches, and the like can contain the following ingredients: a binder, such as microcrystalline cellulose, gum tragacanth or gelatin; an excipient, such as starch or lactose; a disintegrating agent, such as alginic acid, Primolgel, corn starch, and the like; a lubricant, such as magnesium sterate or Sterotes; a glidant, such as colloidal silicon dioxide; a sweetening agent, such as sucrose or saccharin; or flavoring agent, such as peppermint, methyl salicylate, or orange flavoring. When the dosage unit form is a capsule, it can contain, in addition to material of the above type, a liquid carrier, such as a fatty oil.

Other dosage unit forms can contain other materials that modify the physical form of the dosage unit, for example, as coatings. Thus, tablets or pills can be coated with sugar, shellac, or other enteric coating agents. A syrup can contain, in addition to the active compounds, sucrose as a sweetener, and preservatives, dyes, colorings, and flavors. Materials used in preparing these compositions should be pharmaceutically pure and non-toxic in the amounts used.

For purposes of parenteral therapeutic administration, such as by intravenous or intramuscular injection, the xanthines can be incorporated into a solution or suspension. These preparations should contain at least 0.1% of the aforesaid compound, but may be varied between 0.5% and about 50% of the weight thereof. The amount of active compound in such compositions is such that a suitable dosage will be obtained. Preferred compositions and preparations according to the present invention are prepared so that a parenteral dosage unit contains between 0.5 mg to 100 mgs of the active compound.

Solutions or suspensions of the xanthines can also include the following components: a sterile diluent, such as water for injection, saline solution, oils, polyethylene glycols, glycerine, propylene glycol or other synthetic solvents; antibacterial agents, such as benzyl alcohol or methyl parabens; antioxidants, such as ascorbic acid or sodium bisulfite; chelating agents, such as ethylenediaminetetraacetic acid; buffers, such as acetates, citrates, or phosphates; and agents for the adjustment of tonicity, such as sodium chloride or dextrose. The parenteral preparation can be enclosed in ampoules, disposable syringes, or multiple dose vials made of glass or plastic.

While dosage values will vary, good results are achieved when the xanthines of formula (I) or formula (II) are administered to a subject requiring such treatment as an effective oral, parenteral or intravenous dose of from 0.1 to 25 mg/kg of body weight per day. A particularly preferred effective amount is about 1.0 mg/kg of body weight per day. In general, daily dosages will vary from 10-5000 mg, preferably 1600-3200 mg per day.

It is to be understood, however, that for any particular subject, specific dosage regimens should be adjusted to the individual need and the professional judgment of the person administering or supervising the administration of the xanthines. It is to be further understood that the dosages set forth herein are exemplary only and that they do not, to any extent, limit the scope or practice of the invention.

The xanthines are employed before, after, or simultaneously with other treatments that inhibit retroviral activity. In the case of HIV, the xanthines are employed with 3'-azido-2',3'-dideoxythymidine (AZT), 2',3'-dideoxycytidine (ddC), 2',3'-dideoxyadenosine (ddA), 2',3'-dideoxyinosine (ddI), or combinations of these drugs. In addition to the dideoxy nucleosides, a number of other agents can be employed in combination with the xanthines of this invention. For example, the xanthines can be administered in connection with therapies based on the use of the compounds HPA 23, phosphonoformate (foscarnet), ribavirin, and rifabutin. Similarly, the xanthines of this invention can be employed with colony stimulating factors, such as granulocyte colony stimulating factor (G-CSF), granulocyte macrophage colony stimulating factor (GM-CSF), or combinations of these drugs. The xanthines can also be employed according to the invention with other drugs, such as acyclovir, α-interferon, or combinations of these drugs with a dideoxynucleoside, such as AZT. The antiviral therapies described herein can also be employed in combination with therapies to boost a subject's immune system. For example, immunostimulation or immunoreconstitution can be achieved by bone marrow transplantation, use of the drug ampligen, or by interleukin-2 administration to a human patient. Furthermore, the xanthines can be employed with other combination therapies, such as AZT in combination with ampligen, ddC, or GM-CSF. The xanthines can also be employed with peptides used to prevent or inhibit HIV infection or replication.

This invention can be employed to treat retroviral infection in a human subject or as a prophylactic for such infection. This invention makes it possible to improve a patient's condition and sense of well being by inhibiting replication of a retrovirus. An increase in patient survival is contemplated.

3. Description and Preparation of Xanthines of Formula (II)

Inhibition of human retrovirus activity can be achieved by administering to a human patient certain antiviral agents in combination with a xanthine of the formula:

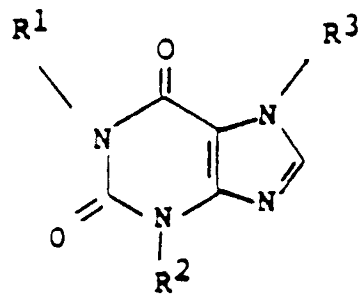

(II)

wherein at least one of $R^1$ and $R^3$ is either

(a) a branched hydroxyalkyl group of the formula

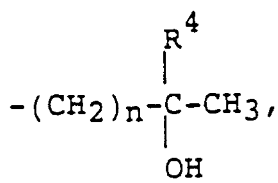

with a tertiary alcohol function, in which $R^4$ stands for an alkyl group with 1 to 3 carbon atoms and n stands for a whole number from 2 to 5, the other $R^1$ or $R^3$ group stands for a hydrogen atom or an aliphatic hydrocarbon group $R^5$ with up to 6 carbon atoms, whose carbon chain may be interrupted by up to 2 oxygen atoms or may be substituted with a hydroxy or oxo group, or

(b) at least one of $R^1$ or $R^3$ is an oxoalkyl group of the formula

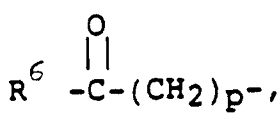

wherein $R^6$ is $C_1$-$C_6$ alkyl, and p = 2, 3 or 4; the other $R^1$ or $R^3$ being defined as above, and $R^2$ represents an alkyl group with 1 to 4 carbon atoms. The xanthine of formula (II) is employed in an amount that is effective in inhibiting retroviral activity. Among these compounds is the commercially available compound pentoxifylline (Trental®). A host of other compounds within the general formula (II) can be employed for inhibiting the activity of human retroviruses. Among these compounds are those identified below.

RETROVIRAL ACTIVITY
INHIBITING
COMPOUNDS OF FORMULA (II)

| Compound Number | $R_1$ | $R_2$ | $R_3$ |
|---|---|---|---|
| 2 | O<br>‖<br>$CH_3-C-(CH_2)_4-$ | $-CH_3$ | $-CH_2-CH_2-CH_3$ |
| 3 | OH<br>\|<br>$CH_3-C-(CH_2)_4-$<br>\|<br>$CH_3$ | $-CH_3$ | $-CH_2-CH_2-O-CH_3$ |
| 4 | " | " | $-CH_2-O-(CH_2)_2-O-CH_3$ |
| 5 | " | " | $-H$ |
| 6 | " | " | $-CH_2-CH_2-CH_3$ |
| 7 | " | " | OH<br>\|<br>$-CH_2-CH-CH_3$ |
| 8 | " | " | OH<br>\|<br>$-CH_2-C-(CH_3)_2$ |
| 9 | " | $-CH_2-CH_3$ | $-CH_2-O-CH_2-CH_3$ |
| 10 | " | $-CH_3$ | $CH_3$<br>\|<br>$-(CH_2)_4-C-CH_3$<br>\|<br>OH |
| 11 | " | " | $-CH_2-O-CH_2-CH_3$ |

The compound 7-ethoxymethyl-1-(5-hydroxy-5-methylhexyl)-3-methyl xanthine, i.e., compound No. 11, is preferred for use in this invention.

The compounds of formula (II) can be prepared according to the disclosure of U.S. Patent 3,737,433 and Belgian Patent 831,051 (where $R^1/R^3$ are oxoallyl). For the case where at least one of $R^1/R^3$ is a tertiary alcohol, reference may be made to International Application PCT/EP86/00401, filed July 8, 1986, claiming German priority of July 8, 1985. This application addresses, as its invention, a variety of embodiments of synthesis routes for the xanthines of formula (II) embraced in the current invention.

An example of one embodiment consists of

a) reacting 3-alkylxanthines of formula (VII)

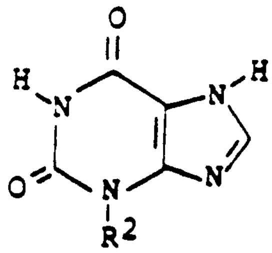

(VII)

in which the $R^3$ represents alkyl with up to 4 carbon atoms, with alkylating agents of formula (VIII)

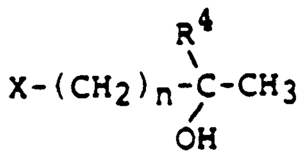

(VIII)

in which X stands for halogen, preferably chlorine, bromine, or iodine, or a sulfonic acid ester group or a phosphoric acid ester group, and wherein $R^4$ and n have the meanings mentioned above, to obtain compounds of formula (IX)

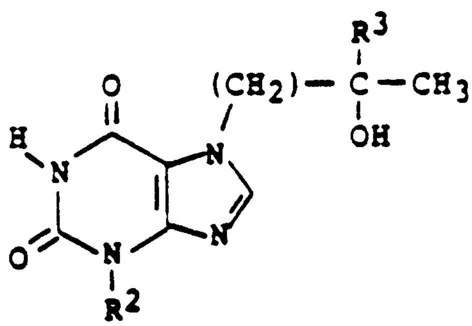

(IX)

with a tertiary hydroxyalkyl group in the position of $R^3$ and hydrogen in the position of $R^1$, and

$a_1$) alkylating this with the same or different alkylating agent of formula (VIII) to obtain compounds pursuant to the invention of formula (X)

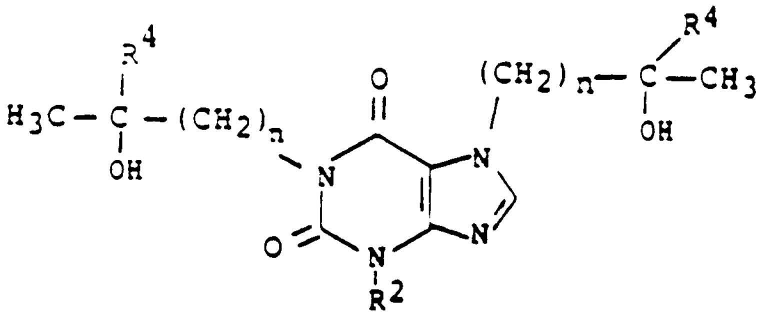

(X)

with two identical or different tertiary hydroxyalkyl groups in the positions of $R^1$ and $R^3$, or
$a_2$) converting it with a compound of the formula

$R^5$-X (Xa)

in which X has the meaning given in formula (VIII) and $R^5$ has the meaning indicated above, into compounds of formula (XI)

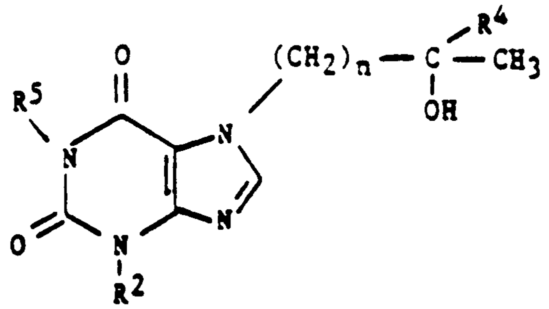

(XI)

in all cases preferably operating in the presence of basic media or using the xanthines in the form of their salts.

Another embodiment consists of
b) substituting 1,3-dialkylated xanthines of formula (XII)

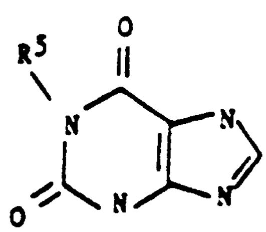

(XII)

in the 7-position, preferably in the presence of basic media or in the form of their salts, by one-step reaction with a compound of formula (VIII), to obtain compounds of formula (XI).

Another embodiment consists of

c) first reacting the 3-alkylxanthines of formula (VII), likewise preferably in the presence of basic media or in the form of their salts, with a compound of the formula

$$R^{15}\text{-}X \qquad (XIII)$$

with the formation of 3,7-disubstituted xanthines of formula (XIV)

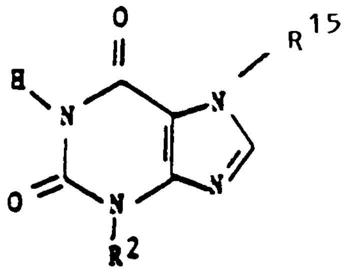

(XIV)

in which $R^{15}$ has the meaning mentioned for $R^5$ or stands for benzyl or diphenylmethyl, and then substituting them in the 1-position, again preferably in the presence of basic media or in the form of their salts, with a compound of formula (VIII). Compounds of formula (XV) are obtained

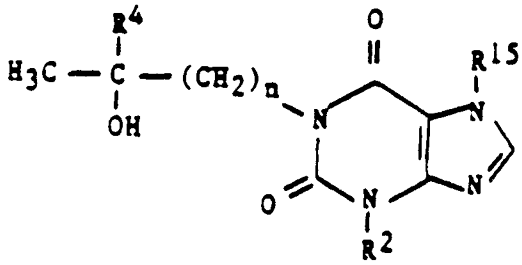

(XV)

in which $R^{15}$ represents a benzyl or diphenylmethyl, and converting the compounds of formula (XV) in which $R^{15}$ represents a benzyl or diphenylmethyl group or an alkoxymethyl or alkoxyalkoxymethyl group, under reducing or hydrolytic conditions, into compounds pursuant to the invention of formula (XVI)

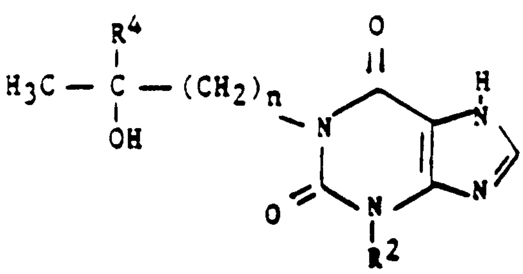

(XVI)

that are subsequently reacted again, if desired, with a compound of formula (VIII) or (Xa) to obtain compounds pursuant to the invention of formula (X) or (XV).

Another embodiment consists of

d) reducing compounds of formula (XI) or (XV) pursuant to the invention in which $R^5$ or $R^{15}$ stands for an oxoalkyl group, with conventional reducing agents for the keto group to obtain the corresponding hydroxyalkylated xanthines pursuant to the invention.

The 3-alkyl- or 1,3-dialkylxanthines of formula (VII) or (XII) used here as starting materials and the "alkylating agents" of formulas (VIII), (Xa), and (XIII) are known for the most part or can be prepared readily by methods disclosed in the literature. Thus, the tertiary alcohols of formula (VIII), for example, can be obtained by organometallic synthesis by reacting the sterically unhindered haloketones of the formula

$$Hal\text{-}(CH_2)_n\text{-}CO\text{-}CH_3 \qquad (XVII)$$

in a so-called synthetic reaction with reductive alkylation of the carbonyl group, with alkylmetal compounds $R^4$-M, especially of magnesium, zinc, or lithium, for example in the form of alkylmagnesium halides $R^4$-MgHal (Grignard compounds) or of the alkyllithium compounds $R^4$-Li, under the usual conditions (for example, see Houben-Weyl, Vol. VI/1 a, Part 2 (1980), pp. 928-40, especially pp. 1021 ff. and 1104-1112). In the same way, a reaction of the haloketones with the formula

$$Hal\text{-}(CH_2)_n\text{-}CO\text{-}R^4 \qquad (XVIII)$$

with methylmagnesium halides or methyllithium likewise leads to the target.

The hydroxyketones corresponding to the formulas (XVII) and (XVIII) can also be converted smoothly into diols with the alkylmetal compounds in the usual way, either directly or with temporary masking of the hydroxy group, for example by acetal formation with 5,6-dihydro-4H-pyran (for example, see Houben-Weyl, Vol. VI/1 a, Part 2 (1980), pp. 1113-1124), from which compounds of formula (VIII) are formed by selective esterification of the terminal primary hydroxyl groups with sulfonyl or phosphoric halides or anhydrides, advantageously in the presence of basic media.

Other possibilities for the synthesis of the tertiary alcohol derivatives of formula (VIII) consist of the monometallation of ω-chloro-1-bromooalkanes to obtain ω-chloroalkylmetal compounds, (Houben-Weyl, Vol. XIII/2 a (1973), pp. 102 and 319) and their subsequent reaction with the ketones $R^4$-CO-$CH_3$, with the extent of by-product formation from the alkanolates formed as intermediates because of their tendency toward ring closure with the elimination of metal salt being minimized by appropriate temperature control, or of using ω-halo-1-alkanols as starting materials, which are metallated in the usual way, preferably in the form of the tetrahydropyranyl-(2) ether or after alkanolate formation of the hydroxy group (MO-$CH_2$)$_n$-Hal) with any desired alkylmetal compound (for example, see Houben-Weyl, Vol. XIII/2 a (1973), p. 113), then reacting them with the ketones $R^4$-CO-$CH_3$ to obtain the diols mentioned in the preceding paragraph (Houben-Weyl, Vol. VI/1 a, Part 2 (1980), p. 1029), and subsequently selectively esterifying the primary hydroxy group with suitable sulfonic or phosphoric acid derivatives.

A convenient access to compounds of formula (VIII) in which $R^4$ represents a methyl group is also available through the reaction of ω-haloalkanoic acid alkyl esters (Hal-($CH_2$)$_n$-COO-alkyl) with two equivalents of a methylmetal compound, with the ester reacting through the ketone to produce the tertiary alcohol with the introduction of two methyl groups (Houben-Weyl, Vol. VI/1 a, Part 2 (1980), pp. 1171-1174). In the same way, ω-hydroxy-carboxylic acid esters can be converted into diols with methylmetal compounds with or without protection of the hydroxy group, for example in the form of tetrahydropyranyl-(2) or methoxymethyl ester, or optionally in the form of the lactones as cyclic esters (for example, see Houben-Weyl, Vol. VI/1 a, Part 2 (1980), pp. 1174-1179), from which active alkylating agents of formula (VIII) can in turn be obtained by selective esterification of the primary hydroxyl group with sulfonic or phosphoric halides or anhydrides.

Suitable compounds of formula (VIII) that can be prepared by the methods described above are thus the [(ω-1)-hydroxy-(ω-1)-methyl]butyl, -pentyl, -hexyl, and -heptyl, the [(ω-2)-hydroxy-(ω-2)-methyl]pentyl, -hexyl, -heptyl, and -octyl, and the [(ω-3)-hydroxy-(ω-3)-methyl]hexyl, -heptyl, -octyl, and -nonyl chlorides, bromides, iodides, sulfonates, and phosphates.

Among the compounds of formula $R^5$-X (Xa) or $R^{15}$-X (XIII) suitable for the introduction of $R^5$ into the 1- or 7-position and of $R^{15}$ into the 7-position of the xanthine skeleton, the alkoxymethyl and alkoxyalkoxymethyl derivatives occupy a special position as their halides can indeed be used successfully as reactants, but toxicological problems can arise, at least in large-scale use. For this reason, the use of the corresponding sulfonates is preferred in this special case, which are readily available, for example, by reacting mixed anhydrides of aliphatic carboxylic acids and aliphatic or aromatic sulfonic acids (M.H. Karger et al., J. Org. Chem. 36 (1971), pp. 528-531) with the formaldehyde dialkyl acetals or dialkoxyalkyl acetals in a smooth and nearly quantitative reaction (M.H. Karger et al., J. Amer. Chem. Soc. 91 (1969), pp. 5663/5665:

$$R^7\text{-}SO_2\text{-}O\text{-}CO\text{-}(C_1\text{-}C_4)Alkyl + R^8\text{-}O\text{-}CH_2\text{-}O\text{-}R^8 \; \text{-}(C_1\text{-}C_4)Alkyl\text{-}CO_2R^8 \rightarrow R^7\text{-}SO_2\text{-}O\text{-}CH_2\text{-}O\text{-}R^8$$

In this equation, $R^7$ represents an aliphatic group such as methyl, ethyl, or trifluoromethyl, or an aromatic group, for example, phenyl, 4-tolyl, or 4-bromophenyl, but preferably methyl or 4-tolyl, and $R^8$ represents an alkyl or alkoxyalkyl group falling under the definition of $R^5$ or $R^{15}$.

The reaction can be carried out either in the substance or in an anhydrous aprotic solvent inert to the reactants at temperatures between -20° and +40°C, preferably between 0° and 20°C. No intermediate isolation of the highly reactive

sulfonates, which are sensitive to hydrolysis and thermally labile, is necessary; they are preferably used immediately as crude products for the substitution on the nitrogen of the xanthines, with the usual addition of a basic condensing agent being unnecessary.

The reaction of the mono- or disubstituted xanthine derivatives, (IX), (XVI), (VII), (VIII) or (Xa) or (XIII) is ordinarily done in a distributing agent or solvent inert to the reactants. Practical representatives are especially dipolar, aprotic solvents, for example formamide, dimethylformamide, dimethyl-acetamide, N-methylpyrrolidone, tetramethylurea, hexamethyl-phosphoric triamide, dimethylsulfoxide, acetone, or butanone; however, alcohols such as methanol, ethylene glycol, and their mono- or dialkyl ethers with the alkyl group having 1 to 4 carbon atoms but both together having a maximum of 5 carbon atoms, ethanol, propanol, isopropanol, and the various butanols; hydrocarbons such as benzene, toluene, or xylenes; halogenated hydrocarbons, such as dichloromethane or chloroform; pyridine, and mixtures of the solvents mentioned or their mixtures with water can also be used.

The "alkylation reactions" are suitably carried out in the presence of a basic condensing agent. Examples of materials suitable for this are alkali metal or alkaline earth hydroxides, carbonates, hydrides, alcoholates, and organic bases, such as trialkylamines (for example, triethyl- or tributylamine), quaternary ammonium or phosphonium hydroxides and crosslinked resins with fixed, optionally substituted ammonium or phosphonium groups. The xanthine derivatives can also be used in the alkylation reaction directly in the form of their separately prepared salts, such as the alkali metal, alkaline earth, or optionally substituted ammonium or phosphonium salts. The mono- and disubstituted xanthine derivatives can also be alkylated either in the presence of the aforementioned inorganic condensing agents or in the form of their alkali metal or alkaline earth salts with the assistance of so-called phase transfer catalysts, for example tertiary amines, quaternary ammonium or phosphonium salts, or crown ethers, preferably in a 2-phase system under the conditions of phase transfer catalysis. Among the suitable phase transfer catalysts that are generally commercially available are tetra ($C_1$-$C_4$)alkyl- and metyltrimethylammonium and -phosphonium salts, methyl-, myristyl-, phenyl-, and benzyltri ($C_1$-$C_4$)alkyl- and cetyltrimethylammonium as well as ($C_1$-$C_{12}$)alkyl- and benzyltriphenylphosphonium salts, with the compounds that have the larger and more symmetrically structured cation generally proving to be the more effective.

The introduction of the groups Ia, $R^5$, and $R^{15}$ by the procedures described above is generally carried out at a reaction temperature between 0°C and the boiling point of the particular reaction medium used, preferably between 20° and 130°, optionally at elevated or reduced pressure, for which the reaction time can amount to less than 1 hour or up to several hours.

The reaction of the 3-alkylxanthines (VIII) to produce the compounds pursuant to the invention of formula (X) requires the introduction of two tertiary hydroxyalkyl groups. Either identical or different substituents can be linked to the xanthine skeleton in succession, or two identical hydroxyalkyl groups can be linked without isolation of intermediates in a single-pot reaction.

The reductive cleavage of the benzyl and diphenylmethyl group from compounds of formula (XV) with the formation of the xanthine atom in the 7-position, is carried out under standard conditions that were developed especially in the framework of the protective group technique in alkaloid and peptide syntheses and can thus be assumed to be widely known. Besides the chemical reduction, particularly of the benzyl compounds with sodium in liquid ammonia (Houben-Weyl, Vol. XI/1 (1957), pp. 974-975), the elimination of the two aforementioned aralkyl groups by catalytic hydrogenolysis using a precious metal catalyst is also especially practical (Houben-Weyl, Vol. XI/1 (1957), pp. 968-971 and Vol. IV//1 c, Part I (1980), pp. 400-404). A lower alcohol is ordinarily used here as the reaction medium (optionally with the addition of formic acid or ammonia), or an aprotic solvent such as dimethylformamide or particularly glacial acetic acid; however, their mixtures with water can also be used. Especially suitable hydrogenation catalysts are palladium black and palladium on activated charcoal or barium sulfate, while other precious metals such as platinum, rhodium, and ruthenium frequently give rise to side reactions because of competitive ring hydrogenation and are therefore only conditionally usable. The hydrogenolysis is preferably carried out at temperatures between 20°C and 100°C and at atmospheric pressure, or preferably slight excess pressure up to approximately 10 bar, with reaction times of a few minutes to several hours generally being needed.

The 1,3,7-trisubstituted xanthines of formula (XV) that have an alkoxymethyl or alkoxyalkoxymethyl group in the position of $R^{15}$ represent O,N-acetals. Consequently, their substituents in the 7-position can be split off under the usual conditions of acid hydrolysis (cf. Houben-Weyl, Vol. VI/I b (1984), pp. 741-745), with the 7H compounds of formula (XVI) likewise being formed. Examples of preferred groups that can be eliminated hydrolytically are the methoxy, ethoxy, and propoxymethyl groups as well as the methoxyethoxy- and ethoxyethoxymethyl groups. The reaction is advantageously carried out with heating in dilute mineral acids such as hydrochloric or sulfuric acid, optionally with the addition of glacial acetic acid, dioxane, tetrahydrofuran, or a lower alcohol as a solution promoter. Also useful are perchloric acid or organic acids, such as trifloroacetic, formic, and acetic acid, in combination with catalytic amounts of mineral acids. The alkoxyalkoxymethyl compounds in particular can also be cleaved by using Lewis acids, such as zinc bromide and titanium tetrachloride in anhydrous medium, preferably in dicholoromethane or chloroform, with the 7-bromomethyl or 7-bromozinc derivatives formed as intermediates hydrolyzing spontaneously during the aqueous workup. In the cleavage in mineral acid solution, the reaction temperature must be chosen so that no significant dehydration of the tertiary hydroxyalkyl group in the 1-position occurs; it should therefore be below 100°C as a rule.

The reduction of the xanthines of formulas (XI) and (XV) with an oxoalkyl group in the position of $R^5$ or $R^{15}$ to the corresponding hydroxyalkyl compounds can indeed take place in principle either with base metals or by catalytic hydrogenation, but the method of choice consists of the reaction occurring under the very mild conditions and in high yields with simple metal hydrides ($MH_n$), complex metal hydrides ($M^1[M^2H_n]_m$), or organometallic hydrides (Houben-Weyl, Vol. IV/1 d (1981), pp. 267-282, and Vol. VI/1 b (1984), pp. 141-155). Of the numerous complex metal hydrides that can be used for the reduction of ketones, the most frequently used reagents might be mentioned, for example, lithium alanate, lithium borohydride, and especially sodium borohydride, that is easier to handle because of its lower reactivity and above all permits working in alcoholic, alcoholic aqueous, and pure aqueous solutions or suspensions. In addition to the otherwise customary inert solvents, such as ethers (for example, diethyl ether, tetrahydrofuran, 1,2-dimethoxyethane), hydrocarbons and pyridine, nitriles, such as acetonitrile, can also be used as the reaction medium. The hydrogenation, which is suitably carried out at temperatures between 0°C and the boiling point of the particular solvent, but preferably at room temperature, generally occurs rapidly and is complete within several minutes to a few hours.

The tertiary hydroxyalkylxanthines of formula (II) can also be prepared by reacting substituted xanthines of formula (XIX)

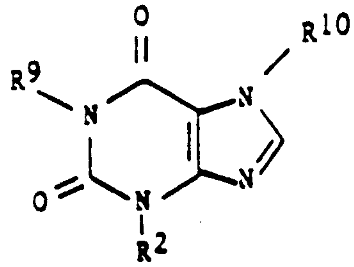

(XIX)

e) contains two identical or different groups of the formula

$$-(CH_2)_n-CO-CH_3 \quad (XX); \text{ or}$$

$$-(CH_2)_n-CO-R^4 \quad (XXI),$$

or only one substituent of the formula (XX) or (XXI), and hydrogen or the group $R^5$ or $R^{15}$ in the positions of $R^9$ and $R^{10}$, with ($C_1$-$C_3$)alkyl- or methylmetal compounds with reductive "alkylation" of the carbonyl groups to obtain the xanthines pursuant to the invention of formulas (IX) to (XVI), or

f) metallating xanthines of formula (XIX) that have two identical or different groups of the formula $-(CH_2)_n$-Hal (XVII), with Hal preferably standing for chlorine or bromine, or only one such group and hydrogen or the substituent $R^5$ or $R^{15}$ in the other position, in the terminal position, and then reacting them with the ketones of the formula

$$R_4-CO-CH_3 \quad (XVIII)$$

with reductive alkylation of the carbonyl group to obtain the xanthines of formulas (IX) to (XVI) pursuant to the invention, or

g) coverting xanthines of formula (XIX) with the group

$$-(CH_2)_n-COO-(C_1-C_4)alkyl \quad (XXIV)$$

in the positions of $R^9$ and/or $R^{10}$ and optionally hydrogen or the group $R^5$ or $R^{15}$ in the other position, by means of two equivalents of a methylmetal compound per alkoxycarbonyl group, into xanthines of formulas (IX) to (XVI) in which $R^4$ stands for methyl, or

h) converting xanthines of formula (XIX) having two identical or different groups of the formula

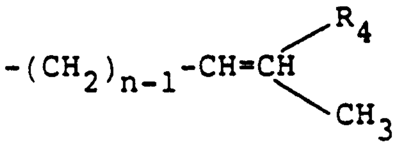

(XXV)

or only one such group and hydrogen or the group $R^5$ or $R^{15}$ in the positions of $R^9$ and $R^{10}$, in which the group (XXV) can contain the C=C double bond also in position-isomeric arrangements on the branched carbon atom, for example, as $-C=CH_2$, by acid-catalyzed hydration obeying the Markownikoff Rule, into the xanthines of formulas (IX) to (XVI) pursuant to the invention, and if desired, then converting the tertiary hydroxyalkylxanthines of formulas Ib' and if obtained pursuant to the invention by methods e) to h) that have a hydrogen atom in the 1- or 7-position, optionally in the presence of basic media or in the form of their salts, with the alkylating agents of formula (VIII) or (Xa) or (XIII), into the trisubstituted compounds of formulas (X) or (XI) or (XV), in which $R^2$, $R^4$, $R^5$, $R^{15}$, and n in the formulas above have the meanings indicated above.

The 3-alkylated mono- or dioxoalkyl- (XIXa), -(ω-haloalkyl) (XIXb), -(ω-alkoxycarbonylalkyl)- (XIXc), and -alkenylxanthines (XIXd) needed for this as starting materials are either known or can be prepared readily, for example, from the 3-alkyl-xanthines (VII) and the sulfonyloxy- or haloketones (XVII) and (XVIII), ω-haloalkylsulfonates, or 1,ω-dihaloalkanes (cf., for example: V.B. Kalcheva et al., Journal fur prakt. Chemie 327 (1985) pp. 165-168), ω-sulfonyloxy or ω-halocarboxylic acid alkyl esters or sulfonyloxy or haloalkenes corresponding to formula (XXV) under the reaction conditions previously described in detail for the alkylation of mono- and disubstitued xanthines with the compounds of formulas (VIII) and (Xa).

In the organometallic reactions of the xanthines (XIXa) and (XIXc) functionalized in the $R^9$ and $R^{10}$ groups, the procedure is the same in principle as described for the preparation of the tertiary alcohols of formula (VIII) used as alkylating agents. Thus, the reductive alkylation of the ketones (XIXa) and of the esters (XIXc) can take place, for example, with alkylpotassium, -sodium, -lithium, -magnesium, -zinc, -cadmium, -aluminum, and -tin compounds. The recently recommended alkyltitanium and -zirconium compounds (D. Seebach et al., Agnew, Chem. 95 (1983), pp. 12-26) can also be used. However, since the alkylmetal compounds of sodium and potassium have a tendency toward side reactions because of their high reactivity and those of zinc and cadmium are relatively sluggish, the alkyllithium and -magnesium (Grignard) compounds are ordinarily preferred.

The strong nucleophilic organometallic compounds are very sensitive to hydrolysis and oxidation. Their safe handling therefore requires working in anhydrous medium, optionally under an inert gas atmosphere. The usual solvents or distributing agents are primarily those that are suitable also for the preparation of the alkylmetal compounds. Practical examples are especially ethers with one or more ether oxygen atoms, for example diethyl, dipropyl, dibutyl, or diisoamyl ether, 1,2-dimethoxyethane, tetrahydrofuran, dioxane, tetrahydropyran, furan, and anisole, and aliphatic or aromatic hydrocarbons, such as petroleum ether, cyclohexane, benzene, toluene, xylenes, diethylbenzenes, and tetrahydronaphthalene; however, tertiary amines, such as triethylamine, or dipolar aprotic solvents, such as hexamethylphosphoric triamide, as well as mixtures of the solvents mentioned can also be used successfully. The reaction of the carbonyl compounds (XIXa) and (XIXc) with the Grignard compounds with the formula $R^4$-MgHal can also beneficially be carried out by placing the organometallic compound in an ether and adding the ketone or the ester dropwise as a solution in dischloromethane or 1,2-dicholoroethane. An addition of magnesium bromide is frequently recommended, which is able to increase the nucleophilicity of the organometallic compound because of its participation in the complex cyclic transition state.

The ketone or ester and the organometallic compound are generally combined at temperatures between -20°C and 100°C, preferably between 0°C and 60°, or at room temperature without external cooling, with the alkylmetal compound ordinarily being used in slight excess. The reaction is then ordinarily completed by brief heating under reflux, for which times of several minutes to a few hours are generally adequate. The alkanolate formed is preferably decomposed with aqueous ammonium chloride solution or dilute acetic acid.

Metallic magnesium and lithium are primarily suitable for the metallation of the ω-haloalkylxanthines (XIXb). On the other hand, the replacement of the halogen atom with lithium, which is also possible using organolithium reagents, generally 1-butyl-, 2-butyl-, t-butyl-, or phenyllithium, plays a subordinate role. However, use is made especially of the Grignard compounds, advantageously preparing them in the ethers, hydrocarbons, tertiary amines, or aprotic solvents listed as particularly suitable for the reaction of the xanthines (XIXa) and (XIXc) with alkylmetal compounds, at temperatures between 25° and 125°C, preferably below 100°C. If the metallation reaction is carried out in hydrocarbons, then

the addition of an ether, such as tetrahydrofuran, or a tertiary amine, such as triethylamine, in stoichiometric amount frequently proves useful. The use of catalysts, such as butanol, aluminum chloride, silicon tetrachloride, tetrachloromethane, and aluminum or magnesium alcoholates, may also be helpful. In the halogen-metal exchange the chlorides ordinarily react more slowly than the corresponding bromides and iodides, but as a rule they provide better yields of organometallic compound. To accelerate the beginning of the reaction, the addition of some magnesium bromide, some grains of iodine, or several drops of bromine, tetrachloromethane, or methyl iodide with slight heating is frequently recommended. The Grignard compounds obtained are normally not isolated, but are reacted immediately with the ketones of formula (XXIII) under the reaction conditions described for the reductive alkylation of the xanthines (XIXa) and (XIXc).

The addition of water to the C=C double bond of the alkenylxanthines (XIXd) with the structural element of formula (XXV), in which the hydroxy group adds to the carbon atom with the fewer hydrogens to form tertiary alcohols according to the Markownikoff Rule, ordinarily occurs in aqueous solution or suspension in the presence of strong acids, such as sulfuric, nitric, or phosphoric acid. Hydrogen halides and sulfonic acids, such as trifluoromethanesulfonic acid, acid exchange resins, boron trifluoride complexes, or oxalic acid, can also be used as catalysts. However, it is preferred to operate in sulfuric acid, with an acid concentration of 50 to 65% and temperatures of 0° to 10°C being sufficient as a rule. However, lower or higher acid concentration and/or reaction temperatures can sometimes also be used. In any case, the reaction temperatures should be kept as low as possible since the reverse dehydration to the olefin can be disturbingly significant above approximately 60°C.

The addition of a solvent inert to acids, such as 1,4-dioxane, benzene, or toluene, sometimes also provides benefits. Since esters can form as intermediates in the acid-catalyzed hydration, particularly when using the high acid concentrations, it is recommended to treat the reaction batch with a large amount of water with brief heating after the action of the acid for the purpose of ester hydrolysis, or to process the mixture in the alkaline range.

The experimental conditions for the optional conversion of the 1- and 7H-compounds (IX) or (XVI) pursuant to the invention into the trisubstituted xanthines of formulas (X) or (XV) by N-alkylation with the compounds (VIII) or (Xa) of (XIII) have already been described above in detail.

Depending on the chain length of the alkyl group $R^4$ (at least $C_2$) and/or the structure of a substituent $R^5$ (for example, 2-hydroxypropyl), the tertiary hydroxyalklyxanthines of formula (II) can have one or two asymmetric carbon atoms and can thus be present in stereoisomeric forms. This invention therefore concerns both the pure stereoisomeric compounds and their mixtures.

This invention will now be described in greater detail in the following Examples.

EXAMPLES

To demonstrate the effectiveness of the claimed invention, a compound of formula (II) was tested to demonstrate inhibition of retroviral activity *in vitro*. Though a variety of compounds within the general formula (II) are effective, they will be exemplified with pentoxifylline as a preferred form of the invention.

EXAMPLE 1

Human Jurkat cells (a $CD4^+$ T-cell lymphoma line) were grown in RPMI-1640 supplemented with 10% fetal calf serum, penicillin, streptomycin and L-glutamine. HIV-1 was produced in Jurkat cells. During the log phase of growth, cell-free supernatant was harvested, tested for RT activity (according to the method of Dayton et al., Cell 1986, **44**:941-947) and frozen in aliquots at -70°C until use. Jurkat cells were pretreated with pentoxifylline at various concentrations for 4 hours after which HIV-1 ($10^4$ cpm units of RT activity) was added to the cultures. The cells remained in the appropriate concentration of pentoxifylline for a total of 7 days, at which point the RT activity was measured in cell-free supernatants. In parallel, cell viability was tested by trypan blue staining of uninfected Jurkat cells treated for 7 days with various concentrations of pentoxifylline. The results are expressed as a percent of control.

| Pentoxifylline (micromolar) | Reverse Transcriptase (% control) |
|---|---|
| 0 | 100 |
| 50 | 84.6 |
| 100 | 72.9 |
| 500 | 69.5 |
| 1000 | 55.0 |

Cell viability after 7 day exposure to pentoxifylline is >95%.

Pentoxifylline decreased HIV-1 replication as measured by reverse transcriptase activity (a marker of HIV-1 replication) in Jurkat cells acutely infected with HIV.

EXAMPLE 2

Human peripheral blood mononuclear cells (PBM) were obtained by Ficoll-Hypaque gradient centrifugation of blood donated by normal HIV-1 sero-negative individuals. PBM were cultured in RPMI-1640 supplemented with 20% fetal calf serum, penicillin, streptomycin and L-glutamine. After stimulating cells overnight with 15 µg/ml of concanavalin A, they were maintained in 10 units/ml interleukin-2 (IL-2) for the duration of the assay. To test for the inhibition of HIV-1 replication in PBM by pentoxifylline, PBM were pretreated with various concentrations of pentoxifylline for 4 hours after which HIV-1 ($10^4$ cpm units of RT activity) was added to the cultures. The cells remained in the appropriate concentrations of the drug for a total of 7 days at which point the RT activity was measured in cell-free supernatants. In parallel, the cytotoxicity was analyzed by trypan blue staining of uninfected PBM treated for 7 days with various concentrations of pentoxifylline. The results are expressed as percent of untreated controls.

| Pentoxifylline (micromolar) | Reverse Transcriptase (% control) | Viability (% control) |
|---|---|---|
| 0 | 100 | 100 |
| 25 | 75.5 | ND |
| 50 | 72.9 | 93.5 |
| 250 | 30.0 | ND |
| 500 | 22.7 | 84.8 |
| 1000 | 18.6 | 50.0 |

Pentoxifylline decreased HIV-1 replication as measured by reverse transcriptase activity in peripheral blood mononuclear cells acutely infected with HIV-1.

EXAMPLE 3

Down-regulation of gene expression mediated by the HIV-1 LTR: U38 cells (Felber BK, Pavlakis GN. Science 1988, **239**:184-187) were cultured in the presence or absence of 10 ng/ml phorbol-12-myristate-13-acetate (PMA). U38 cells were derived from the monocytoid human cell line U937 and contain integrated copies of the HIV-1 LTR ligated to the chloramphenicol acetyl transferase (CAT) gene. These cells have been kindly provided by Dr. Barbara Felber (National Cancer Institute, Frederick, Maryland, USA). Two hours later, the cells were washed once in phosphate-buffered saline (PBS) and cultured in the presence of various concentrations of pentoxifylline. After 2 days, cell viability was tested by trypan blue staining, and the activity of CAT was measured as described previously (Sodroski, J. et al., Science 1985, **227**:171-173) after standardizing the cell extracts for protein content.

| Phorbol ester-stimulated Pentoxifylline (micromolar) | CAT activity |
|---|---|
| 0 | 44.3 |
| 50 | 50.4 |
| 100 | 23.9 |
| 500 | 20.3 |
| 1000 | 19.5 |
| Phorbol ester-non-stimulated Pentoxifylline (micromolar) | CAT activity |
| 0 | 4.75 |
| 50 | 4.91 |
| 100 | 3.75 |
| 500 | 2.95 |
| 1000 | 2.81 |

Pentoxifylline decreased chloramphenicol transacetylase activity (CAT) in U38 cells, whether or not first pretreated with phorbol ester. The CAT assay is frequently used to determine the effects of agents upon a gene activation factor acting on the HIV-1 long terminal repeat (LTR).

In summary, xanthines as hierin described in combination with antiviral agents are especially useful as antiviral agents for the therapeutic treatment of humans. The xanthines are valuable therapeutic agents for both prophylaxis and treatment of retroviral infection in humans. They exhibit antiviral activity against the AIDS viruses, which is highly unusual and unexpected in view of the very limited and specific antiviral activity of the prior art antiviral agents. The xanthines may exhibit suppression of virus-induced cell injury in animal and human cell tissue. The xanthines may also reduce mortality and morbidity manifestations in humans, including a reduction in the occurrence of opportunistic infections associated with AIDS and a reduction in progressive, degenerative effects of HIV on the central nervous system.

## Claims

1. Use of a combined amount of

A) a compound of the formula II

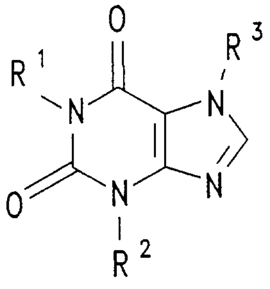

(II)

wherein

a) one of $R^1$ and $R^3$ is a branched hydroxyalkyl group of the formula

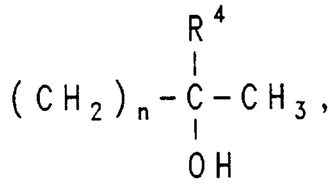

in which $R^4$ stands for an alkyl group with 1 to 3 carbon atoms and n stands for a whole number from 2 to 5, the other $R^1$ or $R^3$ group is a hydrogen atom or an aliphatic hydrocarbon group with up to 6 carbon atoms, whose carbon chain may be interrupted by up to 2 oxygen atoms or may be substituted with a hydroxy or oxo group, or

b) one of $R^1$ and $R^3$ is an oxoalkyl group of the formula

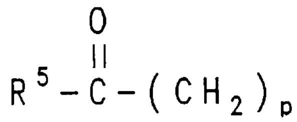

wherein $R^5$ is $C_1$-$C_6$ alkyl and p is 2, 3 or 4, the other $R^1$ or $R^3$ is a hydrogen atom or an aliphatic hydrocarbon group with up to 6 carbon atoms, whose carbon chain may be interrupted by up to 2 oxygen atoms or may be substituted with a hydroxy or oxo group, or

c) $R^1$ and $R^3$ are

I) a branched hydroxyalkyl group of the formula

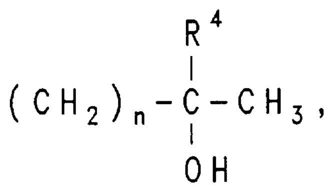

in which $R^4$ is as defined above or

II) an oxoalkyl group of the formula

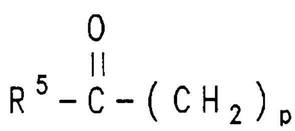

wherein $R^5$ is as defined above;

and $R^2$ is an $C_1$-$C_4$ alkyl group; and

B) an antiviral agent selected from the group consisting of 3'-azido-2',3'-dideoxythymidine (AZT); 2',3'-dideoxycytidine (ddC); 2',3'-dideoxyadenosine (ddA); and 2',3'-dideoxyinosine (ddI); or combinations of these agents;

for the preparation of a medicament effective in inhibiting replication of a human immunodeficiency virus.

2. The use of claim 1, wherein the human is a patient with AIDS.

3. The use of claim 1, wherein a combined amount of pentoxifylline and AZT is administered to a human with AIDS.

4. The use of claim 1, wherein the pentoxifylline is administered in an amount of about 1600-3200 mg/day.

5. The use of claim 1, wherein said combined amount of the xanthine and the antiviral agent is effective in reducing occurrence of opportunistic infections associated with AIDS in the human.

6. The use of a combined amount of

   A) a metabolite of pentoxifylline selected from the group consisting of 1-(5-hydroxyhexyl)-3,7-dimethylxanthine; 1-(5,6-dihydroxyhexyl)-3,7-dimethylxanthine; 1-(4,5-dihydroxyhexyl)-3,7-dimethylxanthine; 1-(4-carboxybutyl)-3,7-dimethylxanthine; 1-(3-carboxypropyl)-3,7-dimethylxanthine; 1-(5-oxohexyl)-3-methylxanthine and 1-(5-hydroxyhexyl)-3-methylxanthine; and

   B) an antiviral agent selected from the group consisting of 3'-azido-2',3'-dideoxythymidine (AZT); 2',3'-dideoxycytidine (ddC); 2',3'-dideoxyadenosine (ddA); and 2',3'-dideoxyinosine (ddI); or combination of these drugs;

   for the preparation of a medicament effective in inhibiting replication of a human immunodeficiency virus.

7. The use of claim 6, wherein the metabolite is 1-(5-hydroxyhexyl)-3,7-dimethylxanthine.

8. The use of claim 6, wherein the metabolite is 1-(3-carboxypropyl)-3,7-dimethylxanthine.

**Patentansprüche**

1. Verwendung einer kombinierten Menge von

   A) einer Verbindung der Formel II

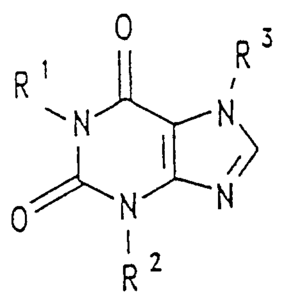

(II),

   worin

   a) einer der Reste $R^1$ und $R^3$ eine verzweigte Hydroxyalkylgruppe mit der Formel

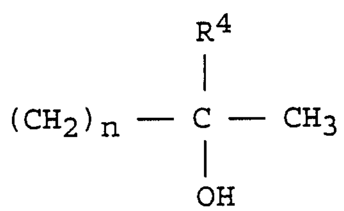

   darstellt, worin $R^4$ für eine Alkylgruppe mit 1 bis 3 Kohlenstoffatomen steht und n eine ganze Zahl von 2

bis 5 bedeutet, während die andere Gruppe $R^1$ oder $R^3$ ein Wasserstoffatom oder eine aliphatische Kohlenwasserstoffgruppe mit bis zu 6 Kohlenstoffatomen bedeutet, deren Kohlenstoffkette durch bis zu 2 Sauerstoffatome unterbrochen sein kann oder durch eine Hydroxy- oder Oxogruppe substituiert sein kann, oder

b) einer der Reste $R^1$ und $R^3$ eine Oxoalkylgruppe mit der Formel

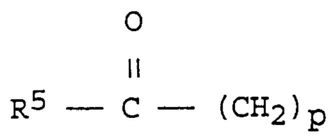

darstellt, worin $R^5$ für $C_1$-$C_6$-Alkyl steht und p den Wert 2, 3 oder 4 aufweist, während der andere Rest $R^1$ oder $R^3$ ein Wasserstoffatom oder eine aliphatische Kohlenwasserstoffgruppe mit bis zu 6 Kohlenstoffatomen bedeutet, deren Kohlenstoffkette durch bis zu 2 Sauerstoffatome unterbrochen sein kann oder durch eine Hydroxy- oder Oxogruppe substituiert sein kann, oder

c) $R^1$ und $R^3$

I) eine verzweigte Hydroxyalkylgruppe der Formel

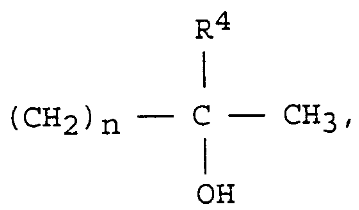

worin $R^4$ wie oben definiert ist, oder

II) eine Oxoalkylgruppe mit der Formel

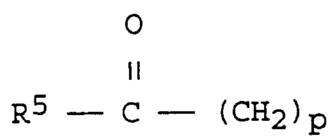

bedeuten, worin $R^5$ wie oben definiert ist;

und $R^2$ eine $C_1$-$C_4$-Alkylgruppe darstellt; und

B) einem antiviralen Mittel, ausgewählt aus der aus 3'-Azido-2',3'-dideoxythymidin (AZT); 2',3'-Dideoxycytidin (ddC); 2',3'-Dideoxyadenosin (ddA); und 2',3'-Dideoxyinosin (ddI); oder Kombinationen dieser Mittel bestehenden Gruppe;

für die Herstellung eines zum Inhibieren der Replikation eines Human-Immundefizienz-Virus wirksamen Medikamentes.

**2.** Verwendung nach Anspruch 1, worin der Mensch ein AIDS-Patient ist.

**3.** Verwendung nach Anspruch 1, worin eine kombinierte Menge von Pentoxifyllin und AZT an einen Menschen mit AIDS verabreicht wird.

**4.** Verwendung nach Anspruch 1, worin das Pentoxifyllin in einer Menge von etwa 1600-3200 mg/Tag verabreicht wird.

5. Verwendung nach Anspruch 1, worin die kombinierte Menge aus dem Xanthin und dem antiviralen Mittel zur Verringerung des Auftretens opportunistischer Infektionen, die mit AIDS beim Menschen einhergehen, wirksam ist.

6. Verwendung einer kombinierten Menge von

A) einem Metaboliten von Pentoxifyllin, ausgewählt aus der aus 1-(5-Hydroxyhexyl)-3,7-dimethylxanthin; 1-(5,6-Dihydroxyhexyl)-3,7-dimethylxanthin; 1-(4,5-Dihydroxyhexyl)-3,7-dimethylxanthin; 1-(4-Carboxybutyl)-3,7-dimethylxanthin; 1-(3-Carboxypropyl)-3,7-dimethylxanthin; 1-(5-Oxohexyl)-3-methylxanthin und 1-(5-Hydroxyhexyl)-3-methylxanthin bestehenden Gruppe, und

B) einem antiviralen Mittel, ausgewählt aus der aus 3'-Azido-2',3'-dideoxythymidin (AZT); 2',3'-Dideoxycytidin (ddC); 2',3'-Dideoxyadenosin (ddA); und 2',3'-Dideoxyinosin (ddI); oder Kombinationen dieser Mittel bestehenden Gruppe;

zur Herstellung eines zur Hemmung der Replikation eines Human-Immundefizienz-Virus wirksamen Medikamentes.

7. 7. Verwendung nach Anspruch 6, worin der Metabolit 1-(5-Hydroxyhexyl)-3,7-dimethylxanthin ist.

8. 8. Verwendung nach Anspruch 6, worin der Metabolit 1-(3-Carboxypropyl)-3,7-dimethylxanthin ist.

**Revendications**

1. Utilisation d'une quantité combinée de

A) un composé de formule II

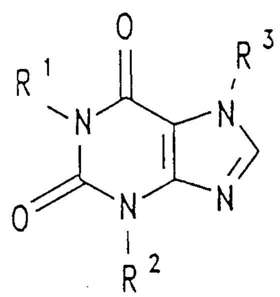

(II)

dans laquelle

a) un de $R^1$ et $R^3$ est un groupe hydroxyalkyle ramifié de formule

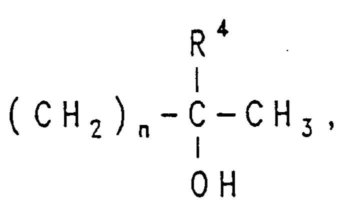

dans laquelle $R^4$ représente un groupe alkyle ayant 1 à 3 atomes de carbone et n est un nombre entier de 2 à 5, l'autre groupe $R^1$ ou $R^3$ est un atome d'hydrogène ou un groupe hydrocarboné aliphatique ayant jusqu'à 6 atomes de carbone, dont la chaîne carbonée peut être interrompue par jusqu'à 2 atomes d'oxygène ou peut être substituée avec un groupe hydroxy ou oxo, ou

b) un de $R^1$ et $R^3$ est un groupe oxoalkyle de formule

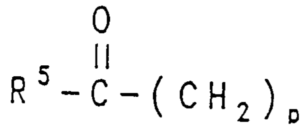

dans laquelle $R^5$ est un groupe alkyle en $C_1$-$C_6$ et p est 2, 3 ou 4, l'autre $R^1$ ou $R^3$ est un atome d'hydrogène ou un groupe hydrocarboné aliphatique ayant jusqu'à 6 atomes de carbone, dont la chaîne carbonée peut être interrompue par jusqu'à 2 atomes d'oxygène ou peut être substituée avec un groupe hydroxy ou oxo, ou

c) $R^1$ et $R^3$ sont

I) un groupe hydroxyalkyle ramifié de formule

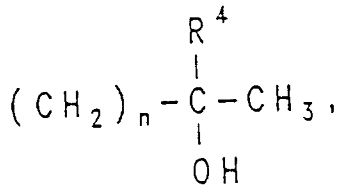

dans laquelle $R^4$ est tel que défini ci-dessus ou

II) un groupe oxoalkyle de formule

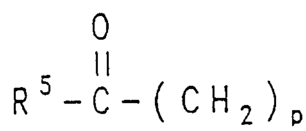

dans laquelle $R^5$ est tel que défini ci-dessus ;

et $R^2$ est un groupe alkyle en $C_1$-$C_4$ ; et

B) un agent antiviral choisi dans le groupe constitué par la 3'-azido-2',3'-didésoxythymidine (AZT) ; la 2',3'-didésoxycytidine (ddC) ; la 2',3'-didésoxyadénosine (ddA) ; et la 2',3'-didésoxyinosine (ddI) ; ou une combinaison de ces agents ;
pour la préparation d'un médicament efficace pour inhiber la réplication d'un virus de l'immunodéficience humaine.

**2.** Utilisation selon la revendication 1, où l'humain est un patient atteint de SIDA.

**3.** Utilisation selon la revendication 1, où une quantité combinée de pentoxifylline et d'AZT est administrée à un humain atteint de SIDA.

**4.** Utilisation selon la revendication 1, où la pentoxifylline est administrée en une quantité d'environ 1 600 à 3 200 mg/j.

**5.** Utilisation selon la revendication 1, où ladite quantité combinée de la xanthine et de l'agent antiviral est efficace pour réduire la survenue d'infections opportunistes associées au SIDA chez l'être humain.

**6.** Utilisation d'une quantité combinée de

A) un métabolite de la pentoxifylline choisi dans le groupe constitué par la 1-(5-hydroxyhexyl)-3,7-diméthylxanthine ; la 1-(5,6-dihydroxyhexyl)-3,7-diméthylxanthine ; la 1-(4,5-dihydroxyhexyl)-3,7-diméthylxanthine ; la 1-(4-carboxybutyl)-3,7-diméthylxanthine ; la 1-(3-carboxypropyl)-3,7-diméthylxanthine ; la 1-(5-oxohexyl)-3-méthylxanthine ; et la 1-(5-hydroxyhexyl)-3-méthylxanthine ; et

B) un agent antiviral choisi dans le groupe constitué par la 3'-azido-2',3'-didésoxythymidine (AZT) ; la 2',3'-didésoxycytidine (ddC) ; la 2',3'-didésoxyadénosine (ddA) ; et la 2',3'-didésoxyinosine (ddI) ; ou une combinaison de ces médicaments ;

pour la préparation d'un médicament efficace pour inhiber la réplication d'un virus de l'immunodéficience humaine.

**7.** Utilisation selon la revendication 6, où le métabolite est la 1-(5-hydroxyhexyl)-3,7-diméthylxanthine.

**8.** Utilisation selon la revendication 6, où le métabolite est la 1-(3-carboxypropyl)-3,7-diméthylxanthine.